# EUROPEAN PATENT SPECIFICATION

(11) **EP 1 816 474 B1**
(45) Date of publication and mention of the grant of the patent: **08.07.2009**
(21) Application number: 05809400.4
(22) Date of filing: 24.11.2005
(51) Int. Cl.: G01N 33/53, A23L 1/30, C08B 37/08

(54) **METHOD OF DETERMINING TROPOMYOSIN IN CHITOSAN**
VERFAHREN ZUR BESTIMMUNG VON TROPOMYOSIN IN CHITOSAN
PROCEDE DESTINE A LA DETERMINATION DE LA TROPOMYOSINE DANS LE CHITOSAN

(30) Priority: 25.11.2004 JP 2004340669
(43) Date of publication of application: 08.08.2007
(73) Proprietor: DAINICHISEIKA COLOR & CHEMICALS MFG. CO. LTD., Tokyo 103-8383 (JP)
(72) Inventor: Kobayashi, Takashi, Dainichiseika Color & Chem.Mfg.Co.,Ltd., Tokyo 103-8383 (JP)
(74) Representative: Hartz, Nikolai
(86) International application number: PCT/JP2005/021597
(87) International publication number: WO 2006/057308

(56) References cited:
- JP-A- 9 110 634
- JP-A- 06 239 902
- JP-A- 09 506 126
- JP-A- 2000 256 196
- JP-A- 2004 346 267
- US-A1- 2004 077 055
- JEOUNG B.J. ET AL.: 'Quantification of the major brown shrimp allergen Pen a 1 (tropomyosin) by a monochlonal antibody based sandwich ELISA.' J. ALLERGY. CLIN. IMMUNOL vol. 100, no. 2, August 1997, pages 229 - 234, XP005189321

## Description

### Technical Field

This invention relates a determination method of tropomyosin in chitosan, and more specifically to a method of determining tropomyosin in chitosan by an immunoassay method, especially an ELISA method. This invention is also concerned with chitosan in which the content of tropomyosin has been assessed by the determination method.

### Background Art

Methods for producing high purity chitosan are known from JP 2004346267 and JP 06-239902. Compositions containing chitosan are known from JP 2000256196 and JP 09110634.
Jeoung et al., J. Allergy Clin. Immunol 100(2). 229-234 (1997) describes quantification of tropomyosin in shrimp meat extracts by ELISA. Chitosan is a functional polysaccharide, and is widely used as a raw material in cosmetics, health foods, feed additives and the like. Most of chitosan available these days on the market in Japan is industrially produced using crab shells as a raw material. Industrially, chitosan is produced as will be described next. Firstly, crab shells discarded at seafood processing factories are collected. The collected crab shells are immersed in dilute hydrochloric acid to convert calcium carbonate into calcium chloride in the crab shells; by washing off the calcium chloride with water, chitin is isolated from the crab shells. The chitin is next immersed in an aqueous solution of sodium hydroxide, said aqueous solution having a high concentration of 40 wt. % or higher, and is then heated to deacetylate chitin into chitosan. The thus-formed chitosan is then thoroughly washed with water to remove excess sodium hydroxide and byproduced sodium acetate, and is subsequently dried to obtain chitosan flakes.

As described above, production steps of chitosan comprise a repetition of immersion steps in an acid and an alkali and a washing step. Further, the deacetylation step of chitin comprises causing chitin to swell with an aqueous solution of sodium hydroxide, said aqueous solution having a high concentration of 40 wt.% or higher, and is conducted until about 80% or more of acetylamino groups in chitin are deacetylated into amino groups. As the thus-formed chitosan is then thoroughly washed with water, proteins in crab shells are hardly believed to remain unmodified, as they are, in chitosan as the final product without being subjected to degradation, to say nothing of proteins adhering on the crab shells.

On the other hand, proteins in chitosan were measured by a conventionally-known protein assay method. Glucosamine units as constituent elements of chitosan, however, acted as an inhibitory factor for the measurement of the proteins, thereby failing to obtain any results worthy for the assessment of the content of the proteins.

### Disclosure of the Invention

### Problems to Be Solved by the Invention

Crab shells, a raw material for chitosan, are known to contain an allergic protein which may develop a food allergy. As mentioned above, it is the common knowledge of these days that the proteins in crab shells are hardly believed to remain unmodified, as they are, in chitosan as the final product without being subjected to degradation, to say nothing of the proteins adhering on the crab shells. Accordingly, the allergic protein is hardly believed to remain as it is in chitosan produced by a method known per se in the art.

Chitosan-containing health foods are widely consumed these days. No allergy problem has, however, taken place to date by the consumption of chitosan along with foods owing to the specific indication of crab shells as a chitosan source, coupled with the setting of a one-gram upper limit on the daily ingestion of chitosan in the health foods.

Under the circumstances that difficulties exist in measuring the amount of a protein in chitosan, especially the amount of an allergic protein or the amount of a peptide fragment (hereinafter simply called "peptide") as a portion of the protein or a residual group of an amino acid constituting the protein, it is meaningful to measure the content of a protein, which has a potential relevance to the development of an allergy, and a content of its peptide and to assess chitosan, which is to be put on the market, by using these contents as an indication of a risk of developing the allergy even if the contents should be totally or substantially irrelevant to the severity of the allergy, and it is also important to produce chitosan low in the above-described contents and to put the chitosan on the market after its assessment in the above-described contents.

The accuracy of an amino acid analysis has been remarkably improved recently; and with a sophisticated new analyzer, it has become possible to quantitate amino acids in chitosan without being affected by interfering factors such as the above-described glucosamine units. It is, however, only the amino acids that can be analyzed here. It is impossible to ascertain whether the above-mentioned allergic protein exists as a monomeric protein or as an oligomer in chitosan, or whether the protein has been degraded to exist as a peptide or as an amino acid. Moreover, such an analyzer is very expensive and therefore, is not suited for widespread use.

An object of the present invention is, therefore, to provide a method for easily determining with high accuracy a protein in chitosan, said protein having a potential relevance to the development of an allergy, specifically tropomyosin and its peptides; and also chitosan having a measurement value not higher than a predetermined value, with the measurement value being assessed to have only a low risk of inducing the allergy.

### Means for Resolving the Problems

Tropomyosin is a muscle protein in crustaceans, is composed of subunits having a molecular weight of about 33,000, and has already been confirmed to be a primary allergen in shrimps. As a result of molecular cloning experiments of lobster's and crab's tropomyosin molecules, this protein is considered to be an allergen common to crustaceans. The measurement of tropomyosin in general foods is, therefore, important for the obviation of food allergy accidents. For this measurement, a method actually making use of an immunoreaction (an enzyme-linked immunosorbent assay (ELISA) method)) has been developed and put into practical use.

The present inventor, therefore, made an investigation to determine whether or nor the ELISA method developed for general foods to measure tropomyosin would be applicable for the measurement of tropomyosin in chitosan. As a result, it has been found that tropomyosin in chitosan can be measured by dissolving solid chitosan in an aqueous solution containing at least one organic acid or the like having chitosan-dissolving power such as acetic acid, lactic acid or pyrrolidonecarboxylic acid to obtain the thus-prepared aqueous solution as a sample to be measured, and then using an ELISA method that uses various antibodies. It has also been found that the amounts of tropomyosin and its peptide remaining in chitosan can be assessed by the above-described measuring method and also that chitosan with tropomyosin and its peptide remaining as low as 100 ppm or lower therein has a remarkably low risk of developing an allergy or is free of such a risk.

Therefore, the present invention provides a method of determining tropomyosin in chitosan, which comprises performing the determination by an immunoassay method, with the chitosan being in a state dissolved in an aqueous solution of a specific organic acid as defined by claim 1.
In the above-described determination method, it is preferred that the immunoassay method is an ELISA method; that the ELISA method is a sandwich technique making use of at least one anti-crustacean tropomyosin antibody as a primary antibody and at least one labeled antibody; and/or that the labeled antibody is an enzyme-labeled anti-crustacean tropomyosin polyclonal antibody or an enzyme-labeled anti-immunoglobulin antibody.

In the above-described determination method, it is preferred that the crustacean is a shrimp and the enzyme is at least one enzyme selected from the group consisting of peroxidase, alkaline phosphatase and β-galactosidase; that the anti-crustacean tropomyosin antibody is coated on an inner wall of a container for determination; and/or that the enzyme is peroxidase and as a substrate for peroxidase, at least one substrate selected from o-phenylenediamine, diammonium 2,2'-azino-bis(3-ethylbenzothiazolinesulfonate) or tetramethylbenzidine is used.

In the above-described method, it is preferred that the determination of tropomyosin is performed by measuring a blue color in a determination system; that the determination of tropomyosin is performed by adding sulfuric acid or phosphoric acid to a determination system, which produces a blue color, to change the determination system into a yellow color and then measuring the yellow color; and/or a concentration of the organic acid in the aqueous solution of the organic acid is 0.1 wt.% or higher but lower than 1 wt.%.

In the above-described method, it is preferred that the chitosan is chitosan having a content of insolubles not higher than 1.0 wt.% as measured by a measuring method which comprises:
(1) drying chitosan at 105°C for 3 hours,
(2) calculating a purity of the chitosan from its weight ratio before and after the drying,
(3) dissolving the pre-drying chitosan in a 1 wt.% aqueous solution of acetic acid to give a pure chitosan (A gram) concentration of 0.5 wt.%,
(4) filtering the resultant aqueous solution through a G3 glass filter (B grams) which has been dried to a constant weight,
(5) washing with distilled water a filtration residue on the filter,
(6) drying the filter and the filtration residue contained thereon at 105°C for 3 hours, and conducting weighing (C grams), and
(7) calculating the content (wt.%) of insolubles in the chitosan in accordance with an equation [(C-B) /A × 100].

A chitosan having a tropomyosin content of 100 ppm or lower (a measurement value obtained by the above-described determination method of the present invention) is also disclosed

### Advantageous Effects of the Invention

According to the present invention, tropomyosin in chitosan can be easily and accurately determined. This determination makes it possible to assess that the risk of development of an allergic reaction to man by the chitosan is significantly low or is none. The present invention can also provide the chitosan assessed to have a significantly low or no risk of the allergic reaction.

### Best Modes for Carrying out the Invention

Before describing the present invention, a description will be made of terms to be used in the present invention.
The term "determination" should include not only the quantitation but also the mere detection of tropomyosin.
The term "primary antibody" should mean an anti-antigen antibody, that is, an antibody to an antigen. This primary antibody should be interpreted to include not only a monoclonal antibody but also a polyclonal antibody. A primary antibody may be or may not be labeled. As an illustrative primary antibody, an anti-crustacean tropomyosin antibody can be mentioned. As an organism species that produces a primary antibody, various organism species commonly used in the present field of art can be used: Such organism species also include cultured cells such as hybridomas.

The term "secondary antibody" means an anti-immunoglobulin antibody, that is, an antibody against an antibody as an antigen. The term "secondary antibody" as used herein, therefore, means not only an antibody against an antibody specific to an antigen but also an immunoglobulin (for example, IgG) itself of an organism which has produced the antigen-specific antibody, that is, an antibody which can bind specifically to IgG of an organism which has produced the antigen-specific antibody. Except for the above-mentioned feature, a secondary antibody is similar to a primary antibody. This secondary antibody may or may not be labeled.

The term "tropomyosin" should be interpreted to embrace, in addition to tropomyosin itself, a peptide as a part of tropomyosin, and "tropomyosin or its peptide" may hereinafter be simply called "tropomyosin" for the sake of brevity.
Other terms have similar meanings as they are currently used in the fields of immunology, molecular biology and biochemistry, unless otherwise specifically indicated.

The present invention will hereinafter be described in further detail based on best modes for carrying out the present invention.
The present invention relates to a method of determining tropomyosin in chitosan. This chitosan is produced using chitin isolated from a crustacean as a raw material. As an isolationmethod of chitin from a crustacean, the Hackman method based on such acid treatment and alkali treatment as described above is common. Without being limited to this method, however, it is possible to use any method developed to date such as a method making use of an enzyme such as protease instead of the alkali treatment

Chitosan can be obtained by deacetylating the isolated chitin in an aqueous solution of an alkali hydroxide. As the alkali hydroxide, sodium hydroxide, potassium hydroxide or the like can be used. The aqueous solution of the alkali hydroxide may preferably have a concentration of 35 wt.% or higher. Although no limitation is imposed on the deacetylation temperature, it may be 20°C or higher, preferably 50°C or higher because a low temperature results in an unrealistic reaction velocity.

Subsequent to the deacetylation of chitin, the resultant chitosan is thoroughly washed with water. As chitosan for use in the method of the present invention, it is preferred to employ, for example, chitosan having insolubles at a content of 1 wt.% or lower when the chitosan is dissolved at a concentration of 0.5 wt. % in a 1 wt. % aqueous solution of acetic acid. No limitation is imposed on the degree of deacetylation of chitosan insofar as such chitosan is employed.

To obtain chitosan with insolubles at such a low content as described above, a degree of deacetylation as high as 70% or even higher is needed in the case of chitosan to be obtained by the heterogeneous deacetylation process (the process that chitin is deacetylated by immersing it in an aqueous solution of an alkali hydroxide without dissolving the same) which is an industrial production process commonly employed at present. In the case of chitosan to be obtained by the homogeneous deacetylation process (the process that chitin is deacetylated in a dissolved state), a degree of deacetylation of 30% or higher is needed. It is to be noted that a degree of deacetylation is a molar fraction of glucosamine units in chitosan--constituting saccharide units as determined by colloid titration. No limitation is imposed on the molecular weight of chitosan in the present invention.

Chitosan, the tropomyosin of which is to be determined, in the present invention is used in a state dissolved in an aqueous solution of an organic acid. However, chitosan is a hardly-dispersible polymer and, when chitosan is in a dispersed state, the state of dispersion of chitosan tends to be insufficient, thereby undesirably leading to a risk of developing an error in the determination results of tropomyosin. Upon determination of tropomyosin in chitosan, it is, therefore, preferred to determine tropomyosin in chitosan after bringing the chitosan, which is to be subjected to the determination, into the state of an aqueous solution by adding an aqueous solution of an organic acid to the chitosan or adding the chitosan to the aqueous solution of the organic acid.

As an organic acid used in the present invention for dissolving chitosan which is a target substance to be determined, acetic acid, lactic acid, pyrrolidonecarboxylic acid are essential. In an aqueous solution of such an organic acid, the concentration of the acid may desirably be 0.1 wt.% or higher but lower than 2 wt.%, with 0.5 wt.% or higher but not higher than 1 wt.% being more preferred. Use of an inorganic acid such as hydrochloric acid instead of the above-described organic acid or, even in the case of an organic acid, an acid concentration of 2 wt.% or higher is not preferred, because the resulting aqueous solution of chitosan is provided with an excessively low pH, so that a greater variation tends to occur in the measurement value of tropomyosin in the aqueous solution of chitosan. Chitosan is, however, not dissolved sufficiently if the concentration of the organic acid is lower than 0.1 wt. %. The above-described organic acid may be used in the form of a salt with chitosan. In this case, the organic acid salt of chitosan may be dissolved in a sample preparation solvent instead of dissolving chitosan in the above-described aqueous solution of the organic acid.

The concentration of chitosan in its aqueous solution to be used in the determination cannot be necessarily specified, because it relies upon the concentration of the organic acid in its aqueous solution to be used. Nonetheless, a chitosan concentration of 0.5 wt. % or higher but not higher than 1 wt.% can be exemplified when the concentration of the aqueous solution of the organic acid is 1 wt.%. This concentration is the concentration before dilution with an extraction buffer or the like.

In the method of the present invention, chitosan which is to be subjected to the determination of tropomyosin may be used preferably in the form of an aqueous solution dissolved in an aqueous solution of an organic acid as described above. Insofar as chitosan is used in the form of such an aqueous solution, no particular limitation is imposed on the manner of determination of tropomyosin in the chitosan provided that the determination is effected by an immunoassay method.

Concerning chitosan to be used, no particular limitations are imposed on its deacetylation degree and molecular weight. Nonetheless, suited is, for example, chitosan the content of insolubles in which is 1 wt.% or lower when the chitosan is dissolved at a concentration of 0.5 wt.% in a 1 wt.% aqueous solution of acetic acid. The insolubles in chitosan are measured by:
(1) drying chitosan at 105°C for 3 hours,
(2) calculating a purity of the chitosan from its weight ratio before and after the drying,
(3) dissolving the pre-drying chitosan in a 1 wt.% aqueous solution of acetic acid to give a pure chitosan (A gram) concentration of 0.5 wt.%,
(4) filtering the resultant aqueous solution through a G3 glass filter (B grams) which has been dried to a constant weight,
(5) washing with distilled water a filtration residue on the filter,
(6) drying the filter and the filtration residue contained thereon at 105°C for 3 hours, and conducting weighing (C grams), and
(7) calculating the content (wt.%) of insolubles in the chitosan in accordance with an equation [(C-B) /A X 100].

The present invention relates to a method of determining tropomyosin (which may hereinafter be called "the antigen" unless otherwise specifically indicated) in the above-described chitosan by using an immunoassaymethod. The immunoassay method includes two techniques, one being a competitive immunoassay technique and the other a sandwich technique. These techniques are both suitably usable in the present invention. In these techniques, it is known to use, as a determination indicator, an isotope (¹³¹I, ¹²⁵I, ¹⁴C, ³H, ⁵⁷Co, ⁷⁵Se or the like), a phosphor, an enzyme or the like and to label an antigen or antibody with such a determination indicator. In the present invention, either an antigen or an antibody can be labeled.

When the sandwich technique is used as a determination technique in the present invention, a technique that makes use of an enzyme-labeled antibody (called "ELISA") is more suited from the standpoints of determination accuracy and readiness. As will be described subsequently herein, the sandwich technique can be practiced by a procedure that forms a conjugated substance (which may also be called "a complex substance") of a primary antibody, an antigen and a labeled (primary or secondary) antibody or by a procedure that forms a conjugated substance of a primary antibody, an antigen, an unlabeled (primary or secondary) antibody and a labeled secondary antibody. These procedures are both suitably usable in the present invention.

In the present invention, tropomyosin to be used as an antigen may preferably be one derived from a crustacean in particular. Illustrative can be tropomyosin derived from crabs, lobsters, shrimps, dephnias, acorn barnacles, mysids, sow bugs or the like, with tropomyosin derived from lobsters, shrimps or crabs being more preferred.

The antibody for use in the present invention can be prepared preferably by using an animal such as mouse, hamster, fowl, goat, rat, rabbit or the like immunized (sensitized) with an antigen, or cells isolated from the animal; or cells (hybridomas) obtained by fusing cancer cells (for example, myeloma) with lymphocytes isolated from the spleen of an animal immunized using an antigen. It is, however, to be noted that the antibody shall not be limited to such examples in the present invention. In other words, any antibody derived from any animal or cells is usable insofar as the object of the present invention can be achieved. A monoclonal antibody may preferably be prepared by using hybridomas.

### 1. Procedure for the formation of a conjugated substance (which may also be called "a complex substance") of a primary antibody, an antigen and a labeled (primary or secondary) antibody

The primary antibody useful in this procedure is at least one primary antibody. This primary antibody may or may not be immobilized (in other words, insolubilized or coated). As the present invention relates to a method of determining tropomyosin in chitosan, the primary antibody can be either a polyclonal antibody or a monoclonal antibody insofar as it is an antibody against tropomyosin. From the standpoint of determination accuracy, however, it is more suited to use a monoclonal antibody as the primary antibody.

The labeled antibody for use in the above-described procedure includes at least one primary antibody or at least one secondary antibody, or a mixture thereof. Similar to the foregoing, these antibodies can be either monoclonal antibodies or polyclonal antibodies. Specific examples of more preferred antibodies include an anti-shrimp tropomyosin antibody as a primary antibody, and a peroxidase-labeled anti-immunoglobulin antibody or peroxidase-labeled anti-shrimp tropomyosin polyclonal antibody as a labeled antibody. It is, however, to be noted that the present invention shall not be limited to the use of these exemplified antibodies.

When an immobilized antibody is used in the above-described procedure, the immobilization of the antibody is conducted to glass, plastics, paper or the like. Specifically, it is preferred to conduct the immobilization of the antibody to a determination container made of glass, plastics, paper or the like, that is, a test tube, small centrifuge tube, a plate having wells (for example, microtiter plate) or beads. The immobilization of the antibody can be conducted in a manner known *per se* in the art. The preparation of the above-described immobilized antibody and labeled antibody can be suitably effected by immunizing a different animal although they can be prepared by immunizing the same animal. In the above-described procedure, the antigen is held between the primary antibody and the labeled antibody to form a sandwich-like conjugated substance.

When a phosphor is used as a labeling substance in the present invention, any phosphor is usable insofar as the object of the present invention can be achieved. Examples include fluorescein isothiocyanate, rhodamine isothiocyanate and phycoerythrin. The labeling of an antibody with a phosphor can be achieved in a manner known per se in the art. The method using an enzyme as a labeling substance is called an ELISA method. Preferred examples of the enzyme to be used in the ELISAmethod include peroxidase, alkaline phosphatase and β-galactosidase from the standpoints of determination accuracy, readiness, etc. Of these, peroxidase is particularly preferred from the standpoint of determination accuracy.

Preferred examples of substrates for the above-described enzymes will be described hereinafter. When the enzyme is peroxidase, o-phenylenediamine (color development: liver brown), diammonium 2,2'-azino-bis(3-ethyl-benzothiazolinesulfonate), tetramethylbenzidine (3,3',5,5'-tetramethylbenzidine) (color development: blue), ABTS (diammonium 2,2'-azino-bis(3-ethylbenzothiazoline-6-sulfate) (color development: blue), and the like can be exemplified. Of these, tetramethylbenzidine is more preferred for its color development sensitivity. When the enzyme is alkaline phosphatase, p-nitrophenylphosphate salts (color development: yellow) can be exemplified. When the enzyme is β-galactosidase, o-nitrophenylphosphoric acid (color development: yellow) can be exemplified. It is, however, to be noted that the present invention shall not be limited to the use of the above-exemplified substrates.

As a method for labeling an antibody with an enzyme, conventionally-known methods are all suitably applicable. As a specific example of the labeling method, more preferred is firstly to biotinylate an antibody (into a biotinylated antibody), to label an enzyme with streptavidin, and then to use the binding between the biotin in the biotinylated antibody and the streptavidin. The enzyme reaction can be conducted under conditions known per se in the art.

### 2. Procedure for the formation of a conjugated substance of a primary antibody, an antigen, an unlabeled (primary or secondary) antibody and a labeled secondary antibody

This procedure uses an unlabeled primary or secondary antibody instead of the labeled antibody in the above-described procedure 1, and additionally uses a labeled secondary antibody. The labeled secondary antibody may be an antibody against the above-described unlabeled antibody (i.e., anti-immunoglobulin) or an antibody specifically binding to the above-described unlabeled antibody (for example, an antibody specifically binding to an antibody in an animal employed for the preparation of the labeled antibody), and no particular limitation is imposed on its derivation. As the labeled secondary antibody, either a monoclonal antibody or a polyclonal antibody is usable in the present invention, although the use of the polyclonal antibody is preferred. Except for the foregoing, the procedure 2 is similar to the above-described procedure 1.

In the above-described procedure, the primary antibody (which, as in the above-described procedure 1, may preferably be immobilized although it may be in an unimmobilized form) and the antigen bind together, and the unlabeled primary or secondary antibody binds to the conjugated substance. Then, the labeled secondary antibody binds to the resulting conjugated substance. The.labeling substance, labeling method and the like are similar to those described above in connection with the procedure 1.

In the above-described procedure, specific examples of more preferred antibodies include an anti-shrimp tropomyosin antibody as the primary antibody, and a peroxidase-labeled anti-immunoglobulin (polyclonal) antibody as the labeled secondary antibody. It is, however, to be noted that the present invention shall not be limited to the use of these exemplified antibodies.

In the above-described procedures 1 and 2, no particular limitations are imposed on the animals from which the antibodies to be used have been derived. Preferred examples, however, include a mouse or rat-derived antibody as the immobilized antibody and a rabbit or goat-derived antibody as the labeled antibody in the procedure 1; and a mouse or rat-derived antibody as the immobilized antibody, a rabbit or goat-derived antibody as the unlabeled antibody, and a goat or rabbit-derived antibody as the labeled secondary antibody. Determination containers coated with various antibodies, which are useful in the above-described procedures 1 and 2, are available on the market (for example, from ELISA SYSTEMS PTY Ltd. and BETHYL LABORATORIES, INC.), and therefore, use of such determination container is preferred. Further, desired antibodies and measurement containers with such desired antibodies coated thereon may also be obtained by relying upon custom preparation services. For such custom preparation services, TAKARA BIO INC. (Otsu, Japan) can be mentioned as an example.

A description will next be made about certain illustrative common determination procedures for the sandwich technique. However, the present invention is, by no means limited to the use of these examples, and various modifications thereof can also be used. As these procedures are described in widely-read publications, for example, [Experimental Medicine, Extra Issue] "Immunological Protocol" (in Japanese), Compiled by Hiromitsu Nakauchi, Published by YODOSHA CO., Ltd. (2004); "Essential Immunology", 8th, Blackwell (1994); "UltraHigh-sensitivity Enzyme Immunology" (in Japanese), Compiled by Eiji Ishikawa, Japan Scientific Societies Press (JSSP) (1993); etc, the present invention can be practiced by making reference to them for details.

Among sandwich techniques, the technique that makes use of an immobilized antibody will hereinafter be exemplified.
i) The above-described procedure 1: The procedure for forming a conjugated substance of a primary antibody, an antigen and a labeled antibody (primary antibody or secondary antibody) comprises the following steps:
1) The primary antibody is immobilized (coated) on an inner wall or inner walls of a determination container such as a test tube or a plate having wells (for example, a microtiter plate) or on surfaces of beads. As the determination container for the immobilization, a container treated with a highly-concentrated solution of protein for an ELISA method or a conventional cell-cultivation container can be suitably employed.

Firstly, the primary antibody is diluted with a buffer, added into the container, and is allowed stand at room temperature for 30 minutes or longer but not longer than 3 hours and then at a low temperature of 0°C or higher but not higher than 10°C for approximately overnight. Subsequently, the primary antibody solution is removed, a blocking solution (a buffer with BSA dissolved therein) is added as much as needed, and after being allowed to stand in a similar manner as described above, the blocking solution is removed. As a result, the antibody is successfully immobilized.
2) Into the container prepared as described above, the above-described aqueous solution of chitosan to be determined is added, and the aqueous chitosan solution is allowed to stand in a similar manner as described above. If any antigen exists in the aqueous chitosan solution, the antigen binds to the antibody. This reaction may be called "a primary immunoreaction" in some instances. It is to be noted that preferably, the sample to be determined (i.e., the aqueous chitosan solution) is diluted with an antigen-extracting buffer to provide it as plural samples of different concentrations for the determination.
3) After the reaction, a washing buffer is added into the container and is then removed to wash the conjugated substance of the antibody and the antigen.

4) A solution with the labeled (primary or secondary) antibody contained therein is added into the container washed in the above step 3, and is then allowed to stand in a similar manner as described above. As a result, a conjugated substance of the immobilized antibody, the antigen and the labeled antibody is successfully formed. This reaction may be called "a secondary immunoreaction" in some instances.
5) After the reaction in the above-described step 4), a washing buffer is added into the container and is then removed to wash the conjugated substance.
As the buffer, a phosphate buffer (PBS; pH: 6 or higher but not higher than 8; concentration: 2 mM or higher but not higher than 500 mM) can be used.
6) The following sub-steps a) to c) are conducted.
a) The following procedures are conducted when the labeling substance in the labeled antibody is an enzyme.

a-1) To the conjugated substance washed in the step 5), an enzyme-substrate solution is added to conduct an enzyme reaction. Although conditions for the enzyme reaction, such as time and temperature, differ depending on the kind of the labeling enzyme, the enzyme reaction may be conducted under common conditions conventionally known with respect to the enzyme.

a-2) Subsequent to the addition of the enzyme-substrate solution, the absorbance or fluorescence intensity of the solution (reaction mixture) is measured at an appropriate wavelength. In this case, the reaction mixture (which may also be called "the measurement system") may be measured, as it is, with time at the appropriate wavelength. As an alternative, the reaction mixture may be measured after a suitable reaction time, adding a reaction terminating solution such as an acid or azide salt. The method which involves the addition of the reaction terminating solution is a more preferred method for its simplicity. As a suitable acid, phosphoric acid (orthophosphoric acid) or sulfuric acid can be mentioned. When 3,3',5,5'-tetramethylbenzidine is used as an enzyme substrate, the reaction mixture is blue. The addition of an acid to the reaction mixture turns the reaction mixture into a yellow color, which is then measured at an appropriate wavelength.

b) When the labeling substance in the labeled antibody is a phosphor, the fluorescence intensity of the reaction mixture is measured.
c) When the labeling substance in the labeled antibody is an isotope, the isotope activity is measured by a scintillation counter.

ii) The above-described procedure 2: The procedure for forming a conjugated substance of a primary antibody, an antigen, an unlabeled (primary or secondary) antibody and a labeled secondary antibody comprises the following steps:
1) Immobilization (coating) of the primary antibody: Similar to the corresponding step in the above-described procedure i).
2) Primary immunoreaction: Similar to the step 2) in the above-described procedure i).
3) Secondary immunoreaction: To the container washed in the step 2), a solution with the unlabeled (primary or secondary) antibody contained therein is added to form a conjugated substance of the immobilized primary antibody, the antigen and the unlabeled (primary or secondary) antibody.

4) To the container after the reaction in the step 3), a buffer is added, followed by its removal, to wash the conjugated substance of the immobilized antibody and the antigen.
5) To the container after washing in the step 4), a solution with the unlabeled secondary antibody contained therein is added to form a conjugated substance of the immobilized antibody, the antigen, the unlabeled (primary or secondary) antibody and the labeled secondary antibody. This reaction may be called "a tertiary immunoreaction" in some instances.
6) To the container after the reaction in the step 5), a buffer is added, followed by its removal, to wash the conjugated substance.
7) The following step a), b) or c) is conducted:
a) Step when the labeling substance in the labeled antibody is an enzyme: Similar to the sub-step a) in the step 6) of the procedure i).
b) Step when the labeling substance in the labeled antibody is a phosphor: Similar to the sub-step b) in the step 6) of the procedure i).
c) Step when the labeling substance in the labeled antibody is an isotope: Similar to the sub-step c) in the step 6) of the procedure i).

The above-described procedures each uses an immobilized antibody. In a procedure that uses an unimmobilized antibody, on the other hand, it is preferred to separate conjugated substances (complex substances) by centrifugation before a primary reaction, secondary reaction and tertiary reaction, respectively.
In each of the above-described measurements, it is preferred to additionally conduct a measurement on the antigen as a reference in parallel with the measurement of the sample as a measurement target. It is also preferred to conduct measurements on positive controls and a negative control in parallel with the above measurements. It is also possible, for example, to prepare a calibration line based on samples, the concentrations of which are known, prior to an actual measurement and to determine the concentration of a sample from the calibration line. This method makes it possible to obtain a measurement value of higher accuracy.

As a measurement instrument (apparatus) for the above-described measurements, one available on the market for such measurements can be used. For example, a microplate reader or the like can be mentioned. Each measurement can be conducted at an appropriate wavelength.

Measuring reagents are available as a kit on the market. It is, therefore, convenient and preferred to achieve the object of the present invention by using such a commercially-available kit. Illustrative is "Crustacean Tropomyosin Residue" Microwell ELISA Product Code: ESCRUR-48 Analysis Kit (product of ELISA SYSTEMS PTY Ltd., Australia). Such an assaying kit being obtained, tropomyosin is preferably capable of being assayed according to an instruction manual attached to the kit.

A chitosan having a tropomyosin content of 100 ppm or lower when measured by the above-described method of the present invention is disclosed. When employed, for example, as a raw material in foods or as a raw material in cosmetics, this chitosan has either no or extremely low potential risk of developing an allergic phenomenon onman and is high in safety. With chitosan having a tropomyosin content higher than 100 ppm, on the other hand, there is a some concern about an allergic reaction to man.

### Examples

The present invention will next be described specifically based on Examples and Comparative Example, in which the designations of "part" or "parts" and "%" are each on a weight basis unless otherwise specifically indicated.
The tropomyosin measurement method in the following Examples and Comparative Example is an immunoassay method, specifically an ELISA method, more specifically a sandwich measurement technique that uses an anti-mouse shrimp tropomyosin antibody as a primary antibody and an anti-mouse shrimp tropomyosin polyclonal antibody-peroxidase conjugate (peroxidase-labeled anti-mouse shrimp tropomyosin polyclonal antibody) as a labeled antibody.

### Example 1

Using a "Crustacean Tropomyosin Residue" Microwell ELISA Product Code: ESCRUR-48 Analysis Kit 48 (a crab/shrimp/lobster allergen testing kit available from ELISA SYSTEMS PTY Ltd.), the tropomyosin remaining in crab-derived chitosan was measured by the following procedure.
The kit was compounded in accordance with the instruction manual as an attachment to the product. Specifically, a washing buffer concentrate (NaCl-containing phosphate buffer) (25 mL) was poured into deionized water (475 mL), and the resulting mixture was placed as a washing buffer in a washing bottle. Further, the washing buffer concentrate (NaCl-containing phosphate buffer) (25 mL) was also poured into deionized water (475 mL), and the resulting mixture was placed as an antigen-extracting solution in a storage bottle.

From a chitosan sample having a viscosity of 500 mPa·s as measured at 20°C by a rotational viscometer and a deacetylation degree of 90% as measured by colloid titration when formed into an aqueous solution with a chitosan concentration and an acetic acid concentration each controlled at 0.5%, respectively, an aliquot of the chitosan (1 part in terms of pure chitosan) was dissolved in deionized water (99 parts) which contained lactic acid (0.5 part), thereby obtaining a 1% aqueous solution of chitosan. An aliquot (1 part) of the aqueous chitosan solution was added to and mixed with an aliquot (9 parts) of the extracting solution which had been heated to 60°C beforehand, thereby obtaining a 0.1% aqueous solution of chitosan. In the above case, the content of insolubles in the chitosan was 0.5% as measured by the above-described measurement method.
The 0.1% aqueous solution of chitosan was placed for 15 minutes in a water bath of 60°C, and was shaken and mixed for 1 minute at every 5^{th} minute to conduct extraction of tropomyosin. The resulting extract was allowed to stand, and was then filtered through a G3 glass filter. The filtrate was thoroughly mixed and was then provided as a sample for a tropomyosin measurement test.

Each component of the kit was then arranged for use in accordance with the use instructions. Specifically, the whole kit was allowed to become the same temperature as room temperature (20°C or higher but not higher than 25°C) before the use of the kit. A well plate equipped with wells as many as needed for the measurement of the sample and controls was provided, and was then set on a holder. At that time, identification marks were placed in the respective wells to avoid any error in the measurement. The wells were coated with an anti-shrimp tropomyosin antibody.

An aqueous solution of a negative control (tropomyosin concentration: 0 ppm) and aqueous solutions of a positive control (tropomyosin concentrations: 0.05 ppm, 0.10 ppm, 0.25 ppm and 0.50 ppm) were added to their corresponding wells at a rate of 100 µL per well. Further, the sample was also added to its corresponding wells at a rate of 100 µL per well. It is to be noted that the individual controls and sample were measured in two runs.

The holder was caused to slowly slide back and forth for 10 seconds to mix the solutions in the respective wells. Each solution was incubated at room temperature for 30 minutes to induce a primary immunoreaction. Subsequent to the reaction, the solutions in the respective wells were removed, the respective wells were filled with the washing buffer until the washing buffer overflowed, and then, the solutions in the respective wells were completely removed. That operation was repeated five times to perform washing. A solution of a peroxidase-labeled anti-shrimp tropomyosin polyclonal antibody was added to the respective wells at a rate of 100 µL per well. The holder was caused to slowly slide back and forth for 10 seconds to mix the solutions in the respective wells. Each solution was incubated at room temperature for 15 minutes to induce a secondary immunoreaction.

After the reaction, the reaction mixtures in the respective wells were removed, the respective wells were filled with the washing buffer until the washing buffer overflowed, and then, the solutions in the respective wells were completely removed. That operation was repeated five times to perform washing.

An enzyme-substrate solution was added to the respective wells at a rate of 100 µL per well. The holder was caused to slowly slide back and forth for 10 seconds to mix the solutions in the respective wells. Each solution was incubated at room temperature for 10 minutes to conduct a color-developing reaction. At that stage, the aqueous solution of the positive control was colored in blue. A terminating solution (an aqueous solution of orthophosphoric acid) was added to the respective wells at a rate of 100 µL per well. The holder was caused to slowly slide back and forth for 10 seconds to mix the solutions in the respective wells such that the enzyme reaction was terminated. At that stage, the solution colored in blue as described above turned into a yellow color.

The measurement of each absorbance was conducted using a microplate reader ("MPR-A4iII", manufactured by TOSOH CORPORATION). Specifically, the sample was measured at a wavelength of 450 nm while the references were measured at a wavelength of 620 nm. It is to be noted that a value obtained as a result of a measurement at an empty well was assumed to be zero. Further, each measurement was conducted within 30 minutes subsequent to the addition of the terminating solution. Absorbances for the tropomyosin concentrations in the respective controls were plotted to obtain a calibration line. Using that calibration line, the concentrations of tropomyosin in the respective samples were determined from the absorbances of the corresponding samples; the crustacean-derived tropomyosin was quantitated in the respective samples; and determinations were made as to whether or not tropomyosin existed.

The concentration of tropomyosin in the above-described sample, which contained chitosan at a concentration of 0.1% in terms of pure chitosan, was determined to be 0.05 ppm from the calibration line. It was, therefore, possible to confirm that the chitosan sample contains 50 ppm of tropomyosin per gram of pure chitosan. That chitosan sample did not show any significant allergic reaction.

### Example 2

As arrangements for a tropomyosin measurement test, an extracting solution and a washing buffer were compounded as in Example 1. From the pyrrolidonecarboxylate salt of a chitosan sample having a viscosity of 100 mPa·s as measured at 20°C by a rotational viscometer and a deacetylation degree of 78% as measured by colloid titration when formed into an aqueous solution with a chitosan concentration and an acetic acid concentration each adjusted at 0.5%, respectively, an aliquot of the chitosan pyrrolidonecarboxylate (1 part in terms of pure chitosan pyrrolidonecarboxylate) was dissolved in a diluting/extracting solution (99 parts), which had been heated to 60°C, beforehand, to obtain a 1% aqueous solution of chitosan pyrrolidonecarboxylate. In the above case, the content of insolubles in the chitosan was 0.3% as measured by the above-described measurement method.

The aqueous solution was placed for 15 minutes in a water bath of 60°C, and was shaken and mixed for 1 minute at every 5^{th} minute to conduct extraction of tropomyosin. The resulting extract was allowed to stand, and was then filtered through a G3 glass filter. The filtrate was thoroughly mixed and was then provided as a sample for a test. Further, an aliquot (1 part) of the aqueous solution was added to and mixed with an extracting solution (9 parts), which had been heated to 60°C beforehand, to obtain a 0.1% aqueous solution of chitosan pyrrolidonecarboxylate. Subsequent to thorough mixing, the aqueous solution was provided for a test.

The concentration of tropomyosin in the 1% aqueous solution of chitosan pyrrolidonecarboxylate was determined to be 0.5 ppm from the calibration line. It was, therefore, possible to confirm that tropomyosin was contained at 50 ppm per gram of pure chitosan pyrrolidonecarboxylate. Further, the concentration of tropomyosin in the 0.1% aqueous solution of chitosan pyrrolidonecarboxylate was determined to be 0.05 ppm from the calibration line. It was, therefore, possible to confirm that tropomyosin was contained at 50 ppm per gram of pure chitosan pyrrolidonecarboxylate.

As the content of chitosan in chitosan pyrrolidonecarboxylate is 70%, it was possible to confirm that tropomyosin was contained at 71 ppm per gram of pure chitosan pyrrolidonecarboxylate. That chitosan pyrrolidonecarboxylate did not show any significant allergic reaction.

### Example 3

Tropomyosin was measured in a similar manner as in Example 1 except that a 1% aqueous solution of acetic acid was used in place of the aqueous solution of lactic acid and the concentration of chitosan was set at 1%. The same results as in Example 1 were obtained.

### Example 4

From a chitosan sample having a viscosity of 300 mPa·s as measured at 20°C by a rotational viscometer and a deacetylat ion degree of 100% as measured by colloid titration when formed into an aqueous solution with a chitosan concentration and an acetic acid concentration each adjusted at 0.5%, respectively, an aliquot of the chitosan (1 part in terms of pure chitosan) was dissolved in deionized water (99 parts) which contained lactic acid (0.5 part), thereby obtaining a 1% aqueous solution of chitosan. An aliquot (1 part) of the aqueous chitosan solution was added to and mixed with an aliquot (9 parts) of the extracting solution which had been heated to 60°C beforehand, thereby obtaining a 0.1% aqueous solution of chitosan. In the above case, the content of insolubles in the chitosan was 0.2% as measured by the above-described measurement method.
Tropomyosin was measured in a similar manner as in Example 1 except for the use of the above-described chitosan in place of the chitosan in Example 1. The concentration of tropomyosin in the chitosan was lower than the detection limit of the present method. The chitosan sample did not show any significant allergic reaction.

### Comparative Example 1

As arrangements for a tropomyosin measurement test, an extracting solution and a washing buffer were compounded as in Example 1. An aliquot (1 part in terms of pure chitosan) of the chitosan sample used in Example 1 was placed in a beaker in which the extracting solution (9 parts) heated to 60°C beforehand was contained. The resultant solution was mixed by a magnetic stirrer to obtain a 10% dispersion of chitosan. The dispersion was placed for 15 minutes in a water bath of 60°C, and was shaken and mixed for 1 minute at every 5^{th} minute to conduct extraction of tropomyosin. The resulting extract was allowed to stand, and was then filtered through a G3 glass filter. The filtrate was thoroughly mixed and was then provided for a test.

Similarly, an aliquot (1 part in terms of pure chitosan) of the chitosan sample used in Example 1 was added to and mixed with a diluting/extracting solution (99 parts), which had been heated to 60°C beforehand, to obtain a 1% dispersion of chitosan. The dispersion was placed for 15 minutes in a water bath of 60°C, and was shaken and mixed for 1 minute at every 5^{th} minute to conduct extraction of tropomyosin. The resulting extract was allowed to stand, and was then filtered through a G3 glass filter. The filtrate was thoroughly mixed and was then provided for a test.
Further, an aliquot (1 part) of the 1% aqueous solution of chitosan was mixed with an aliquot (9 parts) of the diluting solution, which had been heated to 60°C beforehand, to obtain a 0.1% dispersion of chitosan. Subsequent to thorough mixing, the dispersion was provided for a tropomyosin measuring test.

In a similar manner as in Example 1, measurements were conducted, and based on the measurement results of tropomyosin, determinations were made. No tropomyosin was detected in any one of the chitosan dispersions which contained the chitosan at 10%, 1% and 0.1%, respectively, in terms of pure chitosan. As the detection limit of the present kit was 0.1 ppm, it was possible to conclude that per gram of the chitosan sample in terms of pure chitosan, the content of tropomyosin was 1 ppm or lower in the 10% dispersion of the chitosan, 10 ppm or lower in the 1% dispersion of the chitosan, and 100 ppm or lower in the 0.1% dispersion of the chitosan.

Example 1 and Comparative Example 1 used the same chitosan sample, but the results were significantly different. This difference can be attributed presumably to the use of the chitosan in the measurement after its simple dispersion in Comparative Example 1 as opposed to the use of the chitosan in the measurement after dissolving the same in Example 1, and with mere dispersion to such extent at in Comparative Example 1, the tropomyosin in chitosan may not be considered to be fully extracted. The above difference, therefore, indicates that upon analyzing chitosan, its use in a dissolved form for the measurement is preferred.

### Industrial Applicability

According to the present invention, tropomyosin in chitosan can be easily and accurately determined. This determination makes it possible to assess that the risk of development of an allergic reaction to man by the chitosan is significantly low or is none. The present invention can also provide the chitosan assessed to have a significantly low or no risk of the allergic reaction.

## Claims

1. A method of determining tropomyosin in chitosan, which comprises performing said determination by an immunoassay method with said chitosan being in a state dissolved in an aqueous solution of an organic acid, wherein said organic acid is at least one organic acid selected from the group consisting of acetic acid, lactic acid and pyrrolidonecarboxylic acid.

2. A method according to claim 1, wherein said immunoassay method is an ELISA method.

3. A method according to claim 2, wherein said ELISA is a sandwich technique making use of at least one anti-crustacean tropomyosin antibody as a primary antibody and at least one labeled antibody.

4. A method according to claim 3, wherein said labeled antibody is an enzyme-labeled anti-crustacean tropomyosin polyclonal antibody or an enzyme-labeled anti-immunoglobulin antibody.

5. A method according to claim 4, wherein said crustacean is a shrimp, and said enzyme is at least one enzyme selected from the group consisting of peroxidase, alkaline phosphatase and beta-galactosidase.

6. A method according to claim 3, wherein said anti-crustacean tropomyosin antibody is coated on an inner wall of a container for determination.

7. A method according to claim 4, wherein said enzyme is peroxidase, and as a substrate for peroxidase, at least one substrate selected from o-phenylenediamine, diammonium 2,2'-azino-bis(3-ethylbenzothiazoline-sulfonate) or tetramethylbenzidine is used.

8. A method according to claim 7, wherein said determination of tropomyosin is performed by measuring a blue color in a determination system.

9. A method according to claim 8, wherein said determination of tropomyosin is performed by adding sulfuric acid or phosphoric acid to a determination system, which produces a blue color, to change said determination system into a yellow color and then measuring said yellow color.

10. A method according to claim 1, wherein a concentration of said organic acid in said aqueous solution of said organic acid is 0.1 wt.% or higher but lower than 2 wt.%.

11. A method according to claim 1, wherein said chitosan is chitosan having a content of insolubles not higher than 1.0 wt.% as measured by a measuring method which comprises:
(1) drying chitosan at 105°C for 3 hours,
(2) calculating a purity of said chitosan from its weight ratio before and after said drying,
(3) dissolving said pre-drying chitosan in a 1 wt.% aqueous solution of acetic acid to give a pure chitosan (A gram) concentration of 0.5 wt.%,
(4) filtering the resultant aqueous solution through a G3 glass filter (B grams) which has been dried to a constant weight,
(5) washing with distilled water a filtration residue on said filter,
(6) drying said filter and said filtration residue contained thereon at 105 °C for 3 hours, and conducting weighing (C grams), and
(7) calculating said content (wt.%) of insolubles in said chitosan in accordance with an equation [(C-B)/A X 100].

## Patentansprüche

1. Verfahren zur Bestimmung von Tropomyosin in Chitosan, das eine Durchführung der Bestimmung mit einem Immunoassay-Verfahren umfasst, wobei sich das Chitosan im gelösten Zustand in einer wässrigen Lösung einer organischen Säure befindet, wobei die organische Säure mindestens eine organische Säure ist, die ausgewählt wird aus der Gruppe, bestehend aus Essigsäure, Milchsäure und Pyrrolidoncarbonsäure.

2. Das Verfahren nach Anspruch 1, wobei das Immunoassayverfahren ein ELISA-Verfahren ist.

3. Das Verfahren nach Anspruch 2, wobei es sich bei dem ELISA-Verfahren um eine Sandwich-Technik handelt bei der mindestens ein Antikrebstiertropomyosinantikörper als primärer Antikörper und mindestens ein Antikörper mit einer Markierung verwendet wird.

4. Das Verfahren nach Anspruch 3, wobei der markierte Antikörper ein Enzymmarkierter polyclonaler Antikrebstiertropomyosinantikörper oder ein Enzymmarkierter Antiimmunoglobulinantikörper ist.

5. Das Verfahren nach Anspruch 4, wobei das Krebstier eine Garnele ist, und das Enzym mindestens ein Enzym ist, ausgewählt aus der Gruppe bestehend aus Peroxiasen, alkalischen Phosphatasen und Beta-Galaktosidasen.

6. Das Verfahren gemäß Anspruch 3, wobei der Antikrebstiertropomyosinantikörper auf einer Innenwand eines Behälters für die Bestimmung beschichtet vorliegt.

7. Das Verfahren nach Anspruch 4, wobei das Enzym eine Peroxidase ist und als Peroxidasesubstrat mindestens ein Substrat verwendet wird, das ausgewählt ist aus o-Phenylendiamin, Diammonium 2,2'-azino-bis(3-ethylbenzothiazolinsulfonat) oder Tetramethylbenzidin.

8. Das Verfahren nach Anspruch 7, wobei die Bestimmung von Tropomyosin durchgeführt wird durch Vermessen einer blauen Farbe in einem Bestimmungssystem.

9. Das Verfahren nach Anspruch 8, wobei die Bestimmung von Tropomyosin durchgeführt wird durch Zugabe von Schwefelsäure oder Phosphorsäure zu dem Bestimmungssystem, das eine blaue Farbe erzeugt, sodass das Bestimmungssystem in eine gelbe Farbe umschlägt und anschließend die gelbe Farbe vermessen wird.

10. Das Verfahren nach Anspruch 1, wobei eine Konzentration der organischen Säure in der wässrigen Lösung der organischen Säure 0,1 Gewichtsprozent oder mehr aber weniger als 2 Gewichtsprozent beträgt.

11. Das Verfahren nach Anspruch 1, wobei es sich bei dem Chitosan um ein Chitosan handelt, das einen Gehalt an unlöslichen Stoffen von nicht mehr als 1,0 Gewichtsprozent aufweist nach Messung mit einer Messmethode, die umfasst:
(1) Trocknung von Chitosan bei 105°C für 3 Stunden,
(2) Berechnung einer Reinheit des Chitosan aus dessen Gewichtsverhältnis vor und nach dem Trocknen,
(3) Auflösen des vorgetrockneten Chitosan in einer 1 Gew.-% wässrigen Lösung von Essigsäure, um eine reine Chitosan Konzentration (A Gramm) von 0,5 Gew.-% zu erreichen,
(4) Filtration der erhaltenen wässrigen Lösung durch ein G3-Glasfilter (B Gramm), das auf ein konstantes Gewicht getrocknet wurde,
(5) Waschen eines Filterkuchens mit destilliertem Wasser auf dem Filter,
(6) Trocknung des Filters und des Filterkuchens, der darauf vorliegt, bei 105°C für 3 Stunden und Durchführung eines Abwiegens (C Gramm), und
(7) Berechnung des Gehalts (Gewichtsprozent) der unlöslichen Stoffe in dem Chitosan nach der Formel [(C-B)/A X 100].

## Revendications

1. Procédé pour doser la tropomyosine dans un chitosane, lequel procédé comprend la réalisation dudit dosage par un procédé d'immunoanalyse, ledit chitosane étant dans un état dissous dans une solution aqueuse d'un acide organique, et ledit acide organique étant au moins un acide organique choisi dans le groupe constitué de l'acide acétique, de l'acide lactique et de l'acide pyrrolidonecarboxylique.

2. Procédé selon la revendication 1, dans lequel ledit procédé d'immunoanalyse est un procédé ELISA.

3. Procédé selon la revendication 2, dans lequel ledit procédé ELISA est une technique en sandwich utilisant au moins un anticorps anti-tropomyosine de crustacé en tant qu'anticorps primaire, et au moins un anticorps marqué.

4. Procédé selon la revendication 3, dans lequel ledit anticorps marqué est un anticorps polyclonal anti-tropomyosine de crustacé marqué par une enzyme, ou un anticorps anti-immunoglobuline marqué par une enzyme.

5. Procédé selon la revendication 4, dans lequel ledit crustacé est une crevette, et ladite enzyme est au moins une enzyme choisie dans le groupe constitué de la peroxydase, de la phosphatase alcaline et de la bêta-galactosidase.

6. Procédé selon la revendication 3, dans lequel ledit anticorps anti-tropomyosine de crustacé est déposé sur une paroi intérieure d'un récipient en vue du dosage.

7. Procédé selon la revendication 4, dans lequel ladite enzyme est la peroxydase, et en tant que substrat de la peroxydase, au moins un substrat choisi entre la o-phénylènediamine, le 2,2'-azino-bis(3-éthylbenzothiazoline-sulfonate) de diammonium et la tétraméthylbenzidine, est utilisé.

8. Procédé selon la revendication 7, dans lequel ledit dosage de la tropomyosine est réalisé en mesurant une couleur bleue dans un système de dosage.

9. Procédé selon la revendication 8, dans lequel ledit dosage de la tropomyosine est réalisé en ajoutant de l'acide sulfurique ou de l'acide phosphorique à un système de dosage, qui produit une couleur bleue, afin de changer la couleur du système de dosage en jaune, puis en mesurant ladite couleur jaune.

10. Procédé selon la revendication 1, dans lequel la concentration dudit acide organique dans ladite solution aqueuse d'acide organique est de 0,1 % en poids ou plus, mais inférieure à 2 % en poids.

11. Procédé selon la revendication 1, dans lequel ledit chitosane est un chitosane ayant une teneur en matières insolubles non supérieure à 1,0 % en poids telle que mesurée par un procédé de mesure qui comprend :
(1) le séchage du chitosane à 105 °C pendant 3 heures,
(2) le calcul de la pureté dudit chitosane à partir de son rapport de poids avant et après ledit séchage,
(3) la dissolution dudit chitosane pré-séché dans une solution aqueuse à 1 % en poids d'acide acétique pour obtenir une concentration de chitosane pur (A grammes) de 0,5 % en poids,
(4) la filtration de la solution aqueuse résultante à travers un filtre en verre G3 (B grammes) qui a été séché jusqu'à un poids constant,
(5) le lavage à l'eau distillée du résidu de filtration sur ledit filtre,
(6) le séchage dudit filtre et dudit résidu de filtration présent sur celui-ci à 105 °C pendant 3 heures, et une pesée (C grammes), et
(7) le calcul de ladite teneur (% en poids) en matières insolubles dudit chitosane conformément à l'équation [(C-B)/A × 100].
